# EUROPEAN PATENT SPECIFICATION

(11) **EP 3 718 130 B1**
(45) Date of publication and mention of the grant of the patent: **25.11.2026**
(21) Application number: 18883168.9
(22) Date of filing: 29.11.2018
(51) Int. Cl.: A61L 27/18, A61L 27/54

(54) **ADHESION LAYER BONDED TO AN ACTIVATED SURFACE**
AN EINE AKTIVIERTE OBERFLÄCHE GEBUNDENE HAFTSCHICHT
COUCHE D'ADHÉSION LIÉE À UNE SURFACE ACTIVÉE

(30) Priority: 30.11.2017 US 201762592880 P
(43) Date of publication of application: 07.10.2020
(73) Proprietor: The Trustees of Princeton University, Princeton, NJ 08544 (US)
(72) Inventor: SCHWARTZ, Jeffrey, Princeton NJ 08540 (US); LIM, Kelly, Princeton NJ 08540 (US)
(74) Representative: Hindles Limited
(86) International application number: PCT/US2018/062958
(87) International publication number: WO 2019/108730

(56) References cited:
- WO-A1-2008/075714
- US-A- 5 869 141
- US-A1- 2008 050 598
- US-A1- 2010 068 382
- US-A1- 2010 215 643
- US-A1- 2010 215 643
- US-A1- 2010 323 119
- US-A1- 2014 330 392
- US-A1- 2015 103 504
- US-A1- 2017 044 710
- US-B1- 6 645 644

## Description

### CROSS-REFERENCE TO RELATED APPLICATIONS

This application claims the benefit of U.S. Provisional Application No. 62/592,880, filed on November 30, 2017.

### FIELD OF THE INVENTION

The present invention is related to the field of activation of otherwise unreactive surfaces to make them susceptible to the chemical bonding of coatings, where the coatings allow modification of surface properties. Such coated surfaces have utility as scaffolds for cell growth, reconstructive medicine, and medical devices.

### BACKGROUND

Tissue formation, wound repair, and many disease processes depend on expression and cell-mediated assembly of the appropriate extracellular matrix (ECM) proteins. In particular, oriented ECM fibers are essential for normal tissue development and homeostasis. The organization of the ECM can, however, go awry in many diseases and at sites of injury producing the unaligned collagen fibers that form in scar tissue.

A goal of regenerative medicine is to promote formation of new tissue that closely resembles the normal tissue in organization and function. Controlling cell growth in a spatially defined way enables regeneration of damaged or diseased tissues having the proper alignment of constituent cells and/or alignment of molecular complexes that the cells produce. In particular, cells direct the arrangement of ECM fibrils to correspond to their actin filaments by using cell surface receptors that are indirectly connected to the actin cytoskeleton. Therefore, a major challenge in regenerative medicine is to promote cells to assemble ECM fibrils, such as collagen, into particular orientations or alignments on a scaffold device in order to generate tissues with the required functional properties.

Such a scaffold requires an appropriate substrate surface on which to attach cells in an environment that stimulates the generation of an ECM.

US 2014/330392 A1 describes tissue scaffolds, methods of generating such scaffolds, and methods of use of such scaffolds to generate aligned and functional tissues for use in methods including regenerative medicine, wound repair, and transplantation.

US 6645644 B1 describes ligand-containing coating layers on the surface of hydroxide-bearing substrates comprising transition metal atoms selected from the group consisting of Group IVB, Group VB and Group VIB of the Periodic Chart covalently bonded to the surface hydroxyls of said substrate, wherein each transition metal atom is further covalently bonded to one or more ligands of said coting layer, thereby covalently bonding said coating layer to said substrate. The ligands include inorganic phosphates and organic phosphonates.

US 2010/0215643 A1 describes devices which are functionalized to include surface regions having anti-infective agents. Methods are provided for functionalizing various material surfaces to include active surface regions for binding anti-infective agents. Methods are provided by which anti-infective moieties or agents are bonded to functionalized surfaces.

### SUMMARY OF THE DISCLOSURE

### DEFINITIONS

As used herein, the term "covalent" bond refers to a chemical bond that involves the sharing of electron pairs between atoms. The term "coordinate", "coordinative" or "coordinate covalent" bond denotes a two-center, two-electron covalent bond in which both electrons derive from the same atom, such as the bonding of metal ions to ligands. In contrast "ionic" bonding involves electrostatic attraction between oppositely charged ions, where electrons are not shared, but one or more electrons are localized on one of the atoms (the anion) and removed from the other atom (the cation).

As used herein the term "bonded" means affixed or attached, preferably chemically attached without the use of an adhesive. The chemical bond is preferably a covalent or coordinative attachment.

As used herein the terms "reactive", and "unreactive" refer to the ability of a specific functional group to chemically bond with other functional groups, for example in an inorganic adhesion layer.

As disclosed herein, a number of ranges of values are provided. It is understood that each intervening value, to the tenth of the unit of the lower limit, unless the context clearly dictates otherwise, between the upper and lower limits of that range is also specifically disclosed. Each smaller range between any stated value or intervening value in a stated range and any other stated or intervening value in that stated range is encompassed within the invention. The upper and lower limits of these smaller ranges may independently be included or excluded in the range, and each range where either, neither, or both limits are included in the smaller ranges is also encompassed within the invention, subject to any specifically excluded limit in the stated range. Where the stated range includes one or both of the limits, ranges excluding either or both of those included limits are also included in the invention. The term "about" generally includes up to plus or minus 10% of the indicated number. For example, "about 10%" may indicate a range of 9% to 11%, and "about 20" may mean from 18 to 22. Preferably "about" includes up to plus or minus 6% of the indicated value. Alternatively, "about" includes up to plus or minus 5% of the indicated value. Other meanings of "about" may be apparent from the context, such as rounding off, so, for example "about 1" may also mean from 0.5 to 1.4.

The invention is set out in the appended set of claims.

It has now been discovered that an appropriate substrate surface on which to attach cells in an environment that stimulates the generation of an ECM comprises a polymer surface having appropriate surface functional groups, with a chemically bonded inorganic oxide adhesion layer. A self-assembled monolayer (SAM) of appropriate ligands, chemically bonded to the attached inorganic oxide adhesion layer. These ligands can have surface-modifying properties, and can also support the attachment of cells, providing a suitable environment for generation of an ECM.

There are broad classes of polymers that do not have exposed surface functional groups in sufficient density or sufficient reactivity to form covalent or coordinative bonds with the precursor inorganic alkoxides of the bonded adhesion layers. These classes include various polymer surfaces.

A versatile method has now been discovered that can be effected rapidly on an otherwise unreactive polymer surface, which provides a chemically bonded coating that enables control of the surface's properties.

For example, polymers that are otherwise unreactive or of low reactivity toward the chemical bonding of coatings such as phosphonates or siloxanes, can be activated through oxidation methods, including chemical oxidation using chemical oxidants. Other methods include oxygen plasma discharge and corona discharge. These chemical reagents and methods are able to generate oxy, oxo, carbonyl, carboxylic acid or carboxylate functional groups on the surface. Such functional groups can then react with an inorganic alkoxide (such as a Zr alkoxide or Ti alkoxide) to form a chemically bound inorganic adhesion layer.

Suitable polymers that do not react readily with an inorganic alkoxide to form an adhesion layer include polyalkanes or other polymers such as polysiloxanes, polyalkylarenes, polyolefins, polythiols, and polyphosphines.

One aspect of the invention is directed to a construct comprising a coated activated surface comprising an inorganic oxide adhesion layer chemically bonded to the surface, where the inorganic oxide is selected from the group consisting of the oxides of Ti, Zr, Al, Mg, Si, Zn, Mo, Nb, Ta, Sn, W, and V. Preferably the inorganic adhesion layer of the construct is selected from the group consisting of the oxides of Al, Ti, Zr, Si, Mg and Zn.

The polymers can be selected from polysiloxanes (such as polydimethylsiloxane (PDMS)), polyalkanes, polyalkylarenes, polyaldehydes, polyolefins, polythiols, and polyphosphines.

The activated surface coated with an inorganic oxide further comprises a self-assembled monolayer (SAM) bonded to the adhesion layer, where the SAM is selected from organic compounds comprising a phosphonic, carboxylic, sulfonic, phosphinic, phosphoric, sulfinic, or hydroxamic group. The SAM comprises a self-assembled monolayer of phosphonates (SAMP). The phosphonates can be selected from the group consisting of hydrophobic phosphonates, cell-adhesive phosphonates and phosphonates capable of further metal-catalyzed coupling.

The phosphonates can be selected from the group consisting of phosphonic acids of structure 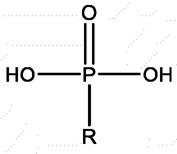 where the R group is selected from the group consisting of optionally substituted alkyl, optionally substituted heteroalkyl, optionally substituted alkenyl, optionally substituted heteroalkenyl, optionally substituted alkynyl, optionally substituted heteroalkynyl, optionally substituted aryl, optionally substituted arylalkyl, optionally substituted heteroaryl, and optionally substituted heteroarylalkyl, where heteroalkyl, heteroalkenyl, heteroalkynyl, heteroaryl and heteroarylalkyl contain one or more heteroatoms selected from the group consisting of O, N and S. Preferably the hydrophobic phosphonates are selected from the group consisting of R = C₃-C₃₀ alkyl. Preferably the cell-adhesive phosphonates are selected from the group consisting of R = C₃-C₃₀ alkyl substituted with a further phosphonate group. More preferably the cell-adhesive phosphonates are selected from the group consisting of C₃-C₃₀ α,ω)-diphosphonates.

Another aspect of the invention is directed to a construct for medical use comprising the inorganic oxide-coated activated surface further containing a SAMP bonded to the inorganic oxide coating. This construct can further comprise other useful moieties covalently bound to the SAMP, such as alkyne or azide groups which allow further elaboration using the so-called "click" reaction, electrochemically active moieties, photochemically active moieties, cell-attractive moieties, cell-adhesive moieties, or anti-infective moieties. Alternatively this medical construct can further comprise cells attached to the SAMP-coated surface. The cells are can be selected from the group consisting of fibroblasts, endothelial cells, keratinocytes, osteoblasts, chondroblasts, chondrocytes, hepatocytes, macrophages, cardiac muscle cells, smooth muscle cells, skeletal muscle cells, tendon cells, ligament cells, epithelial cells, stem cells, neural cells, PC12 cells, neural support cells, Schwann cells, radial glial cells, cells that form neurospheres, neural tumor cells, glioblastoma cells and neuroblastoma cells. The fibroblasts can comprise NIH 3T3 fibroblasts. The construct can further comprise an extracellular matrix (ECM). The construct can be further decelluarized, leaving the ECM attached.

Yet another aspect of the invention is directed to a method of activating and coating an unactivated surface with an inorganic oxide adhesion layer, comprising the steps of: a) activating the surface of an unactivated substrate; b) providing a coating mixture comprising an organic solvent containing an inorganic compound that is reactive with oxy (-O-), oxo (=O), carbonyl (C=O), carboxylic acid (-C(=O)-OH) or carboxylate (-C(=O)-O-) functional groups, and is dissolved and/or dispersed in the solvent; and c) suspending the activated substrate in the coating solution for a time and at a temperature sufficient to form an inorganic oxide coating on the activated surface to provide a coated surface, where the inorganic compound is selected from the group consisting of the alkoxides of Ti, Zr, Al, Mg, Si, Zn, Mo, Nb, Ta, Sn, W, and V. The method can further comprise: d) removing the coated substrate from the coating solution; and e) rinsing with a solvent to provide a rinsed coated substrate. The method can still further comprise: f) heating the rinsed coated substrate to 35 to 40°C.

Alternatively, steps b) and c) can be replaced by vapor deposition of an inorganic alkoxide onto the surface, providing the inorganic oxide adhesion layer.

The inorganic compound of the method can be selected from the group consisting of the alkoxides of Al, Ti, Zr, Si, Mg and Zn. The alkoxide can be selected from the group consisting of methoxide, ethoxide, propoxide, iso-propoxide, butoxide, iso-butoxide, sec-butoxide, and tert-butoxide.

### BRIEF DESCRIPTION OF THE DRAWING

Figure 1 shows the infrared (IR) spectrum of a representative oxygen plasma-oxidized polydimethylsiloxane (PDMS) construct having a titanium iso-propoxide adhesion layer with an octadecylphosphonic acid (ODPA) self-assembled monolayer thereon (Example 1).
Figure 2 displays the elemental composition pattern consistent with the native form of a heparin molecule on a stainless-steel surface (Example 5) as determined by X-ray photon spectroscopy analysis.

### DETAILED DESCRIPTION

The inventive method can be employed on polymer surfaces that are otherwise unreactive with a reactive metal alkoxide. Thus, oxidation of polymers, that are otherwise unreactive toward bonding of coatings, can be activated through oxidation methods including chemical oxidation using chemical oxidants such as permanganate, chlorite, chromic acid, chromate, other chromic acid derivatives, osmium tetroxide, ruthenium tetroxide, iodate, peracids, peroxides, Fenton's reagent (hydrogen peroxide/Fe(II)), lead tetraacetate, lead tetraacetate/Mn(II), ozone, and oxygen. Other methods include oxygen plasma discharge and corona discharge. Oxygen plasma activation can be accomplished as described in US Patent Publication No. 2013/0005660 to Dong et al., or as described in US Patent 9,655,992 to Clevenger et al.. These chemical reagents and methods are able to generate oxy (-O-), oxo (=O), carbonyl (C=O), carboxylic acid (-C(=O)-OH) or carboxylate (-C(=O)-O-) functional groups on the surface. Such functional groups can then react with an inorganic alkoxide (such as a Zr alkoxide or Ti alkoxide) to form a chemically bonded inorganic adhesion layer. Suitable unreactive polymers are unreactive by virtue of lacking appropriate reactive functional groups on the polymer surface, such as the aforesaid oxy, oxo, carbonyl, carboxylic acid or carboxylate functional groups. Suitable unactivated polymers include:
polyalkanes or other polymers with terminal alkyl groups such as polysiloxanes, where C-H bond oxidation would yield C-OH or COOH groups (alcohols or acids) that are reactive;
polyalkylarenes or polyaldehydes, where C-H bond activation would occur oxidatively;
polyolefins, where oxidation would produce glycols;
polythiols, where oxidation would yield sulfonic acids; and
polyphosphines, where oxidation would yield phosphonic, phosphinic or phosphoric acids.

Such activated surfaces provide a platform or substrate on which to construct a chemically bonded adhesion layer which can then be used to attach a self-assembled monolayer (SAM), i.e. a self-assembled monolayer of a phosphonate (SAMP), that would control the surface properties of the material, for example to make it more, or less, hydrophobic, or to attach other useful moieties such as alkyne or azide groups (reactive for "click" chemical coupling), electrochemically active moieties, photochemically active moieties, cell-attractive moieties, cell-adhesive moieties, or anti-infective moieties. Suitable anti-infective moieties are disclosed in the following references: US Patent Publication No. 2010/0215643 to Clevenger et al., US Patent Publication No. 2013/0005660 to Dong et al., and US Patent 9,655,992 to Clevenger et al.

The surface of the substrate comprising a SAM can be patterned or unpatterned.

With regard to the adhesion layer (inorganic oxide coating), the non-oxygen inorganic species preferably has low toxicity in medical applications, and can be advantageously selected from the group consisting of Ti, Zr, Al, Mg, Si, Zn, Mo, Nb, Ta, Sn, W, and V. Preferably the inorganic species is Al, Ti, Zr, Si, Mg or Zn. More preferably the inorganic species is Al, Si, Ti or Zr. The inorganic species can be Al. The inorganic species can be Ti. The inorganic species can be Zr. The inorganic species can be Mg. The inorganic species can be Si. The inorganic species can be Zn. The inorganic species can be Mo. The inorganic species can be Nb. The inorganic species can be Ta. The inorganic species can be Sn. The inorganic species can be W. The inorganic species can be V.

By virtue of its method of synthesis from an inorganic alkoxide, as described herein, the adhesion layer comprises reactive alkoxides on the surface, which reacts with appropriate organic compounds to form a SAMP.

Thus the adhesion layer-coated surface further comprises a self-assembled monolayer (SAM) bonded to the adhesion layer, where the SAM is selected from organic compounds comprising a phosphonic, carboxylic, sulfonic, phosphinic, phosphoric, sulfinic, or hydroxamic group. The SAM comprises a self-assembled monolayer of phosphonates (SAMP). The phosphonates can be selected from hydrophobic phosphonates, cell-adhesive phosphonates and phosphonates capable of further metal-catalyzed coupling. Suitable phosphonates can be selected from the group consisting of phosphonic acids having the structure: 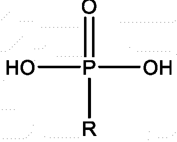 where the R group is selected from the group consisting of optionally substituted alkyl, optionally substituted heteroalkyl, optionally substituted alkenyl, optionally substituted heteroalkenyl, optionally substituted alkynyl, optionally substituted heteroalkynyl, optionally substituted aryl, optionally substituted arylalkyl, optionally substituted heteroaryl, and optionally substituted heteroarylalkyl, where heteroalkyl, heteroalkenyl, heteroalkynyl, heteroaryl and heteroarylalkyl contain one or more heteroatoms selected from the group consisting of O, N and S. The optional substitution on the R group can comprise one or more groups selected from polyol moieties, sugar alcohol moieties, hydroxyl functional groups, amino functional groups, carboxylic acid functional groups, carboxylate ester functional groups, phosphonic acid functional groups, phosphonate functional groups, ether functional groups, alkyne functional groups, azide functional groups and thiol functional groups. Preferably the hydrophobic phosphonates are selected from the group consisting of R = C₃-C₃₀ alkyl. The alkyl group can be a C₅-C₂₄ alkyl, or a C₆-C₂₀ alkyl, or a C₈ to C₁₈ alkyl. The alkyl group can be a C₃, C₄, C₆, C₈, C₁₀, C₁₂, C₁₄, C₁₆, C₁₈, or C₂₀ alkyl group. Preferably the cell-adhesive phosphonates are selected from the group consisting of R = C₃-C₃₀ alkyl substituted with a further phosphonate group. The alkyl group can be a C₄-C₂₄ alkyl, or a C₆-C₂₀ alkyl, or a C₈ to C₁₈ alkyl. The alkyl group can be a C₃, C₄, C₆, C₈, C₁₀, C₁₂, C₁₄, C₁₆, C₁₈, or C₂₀ alkyl group. More preferably the cell-adhesive phosphonates are selected from the group consisting of C₃-C₃₀ ω,ω)-diphosphonates. In this case the alkylene group can be a C₃, C₄, C₆, C₈, C₁₀, C₁₂, C₁₄, C₁₆, C₁₈, C₂₀, C₂₂, C₂₄, C₂₆, C₂₈, or C₃₀ alkylene group. The α,ω-diphosphonic acid can be a C₃₋₁₆ diphosphonic acid, preferably a C₄₋₁₂ diphosphonic acid, more preferably a C₄, or a C₆, or a C₈, or a C₁₀, or a C₁₂ diphosphonic acid. The α,ω-diphosphonic acid can be 1,4-butanediphosphonic acid, or 1,6-hexanediphosphonic acid, or 1,8-octanediphosphonic acid, or 1,10-decanediphosphonic acid, or 1,12-dodecanediphosphonic acid, or mixtures of two or more thereof. Preferably the phosphonates are alkynes, most preferably terminal alkynes. The alkyne phosphonates can have R = C₃-C₃₀ alkynyl. The alkynyl group can be a C₅-C₂₄ alkynyl, or a C₆-C₂₀ alkynyl, or a C₈ to C₁₈ alkynyl. The alkynyl group can be a C₃, C₄, C₆, C₈, C₁₀, C₁₂, C₁₄, C₁₆, C₁₈, or C₂₀ alkynyl group. Preferably the alkynyl group bears a terminal alkyne.

Preferably the SAMP of the coated construct comprises a phosphonic acid covalently attached to the inorganic oxide adhesion layer, which phosphonic acid contains functionality adapted for cell binding. As noted above, the cell-binding phosphonic acid can comprise one or more functional groups selected from polyol moieties, sugar alcohol moieties, hydroxyl functional groups, amino functional groups, carboxylic acid functional groups, carboxylate ester functional groups, phosphonic acid functional groups, phosphonate functional groups, ether functional groups, alkyne functional groups, azide functional groups and thiol functional groups. Preferably the phosphonic acid is a diphosphonic acid, more preferably an α,ω)-diphosphonic acid as described above.

Alkyne and azide functional groups participate in the so-called "click reaction". Click chemistry represents a powerful coupling approach, based on a highly specific and efficient bio-orthogonal reaction between azido- and alkyne-containing compounds, yielding cycloaddition products. The small size and specificity of reaction of the click-reactive groups pre-attached in a SAMP allow facile elaboration of that SAMP to append such desirable moieties as electrochemically active, photochemically active, cell-attractive, cell-adhesive, or anti-infective moieties. Thus, Example 3 describes a copper-catalyzed click coupling of a PDMS/adhesion layer/SAMP of phosphonodec-9-yne with phenyl azide to form a SAMP of terminated with a phenyltriazole.

Click chemistry employs the cycloaddition reaction between a 1,3-dipole and a dipolarophile (specifically azide and alkyne), to form a five-membered ring. The azide and alkyne functional moieties are largely inert toward biological molecules and aqueous environments. Further, the triazole has similarities to the ubiquitous amide moiety found in nature, but unlike an amide, it is not susceptible to cleavage. Additionally, triazoles are not readily oxidized or reduced. Reports and procedures on cycloaddition reactions between a 1,3-dipole and a dipolarophile are readily available to one of ordinary skill in the art. Relevant literature in the field includes Jewett, et al., Chem. Soc. Rev., 2010, 39(4), 1272-1279 and Schultz et al., Org. Lett. 2010, 12(10), 2398-2401.

Further, alkyne groups can participate in various metal-catalyzed coupling reactions, such as palladium-catalyzed coupling reactions, to introduce additional functionality on a SAMP. Reactions such as the Sonogashira reaction are applicable. Thus, Example 2 describes the copper/palladium-catalyzed coupling of a PDMS/adhesion layer/SAMP of phosphonodec-9-yne with bromobenzene to form a SAMP of 10-phenylphosphonodec-9-yne.

Example 4, not according to the invention, describes PDMS coated with an adhesion layer formed from zirconium n-butoxide which can be directly reacted with glycerol, in the absence of a SAM or SAMP.

Another aspect of the invention is directed to a construct for medical applications comprising the coated surface containing a SAMP bonded to the inorganic oxide coating. The construct can further comprise cells attached to the SAMP-coated surface of the construct. The cells can be selected from the group consisting of fibroblasts, endothelial cells, keratinocytes, osteoblasts, chondroblasts, chondrocytes, hepatocytes, macrophages, cardiac muscle cells, smooth muscle cells, skeletal muscle cells, tendon cells, ligament cells, epithelial cells, stem cells, neural cells, PC12 cells, neural support cells, Schwann cells, radial glial cells, cells that form neurospheres, neural tumor cells, glioblastoma cells and neuroblastoma cells. The fibroblasts preferably comprise NIH 3T3 fibroblasts. The construct can further comprise an extracellular matrix (ECM). The ECM is a collection of extracellular molecules secreted by cells that provides structural and biochemical support to the surrounding cells. The construct can be further decelluarized, leaving the ECM attached.

Yet another aspect of the invention is directed to a method of activating and coating an unactivated surface with an inorganic oxide adhesion layer, comprising the steps of: a) activating the surface of an unactivated substrate; b) providing a coating mixture comprising an organic solvent containing an inorganic compound that is reactive withoxy (-O-), oxo (=O), carbonyl (C=O), carboxylic acid (-C(=O)-OH) or carboxylate (-C(=O)-O-) functional groups, and is dissolved and/or dispersed in the solvent; and c) suspending the activated substrate in the coating solution for a time and temperature sufficient to form an inorganic oxide coating on the activated surface to provide a coated surface, where the inorganic compound is selected from the group consisting of the alkoxides of Ti, Zr, Al, Mg, Si, Zn, Mo, Nb, Ta, Sn, W, and V. The method can further comprise: d) removing the coated substrate from the coating solution; and e) rinsing with a solvent to provide a rinsed coated substrate. The method can still further comprise: f) heating the rinsed coated substrate to 35 to 40°C.

Alternatively, steps b) and c) can be replaced by vapor phase deposition of an inorganic alkoxide onto the surface, providing the inorganic oxide adhesion layer.

The inorganic compound of the method can be selected from the group consisting of the alkoxides of Al, Ti, Zr, Si, Mg and Zn. The alkoxide can be selected from the group consisting of methoxide, ethoxide, propoxide, iso-propoxide, butoxide, iso-butoxide, sec-butoxide, and tert-butoxide.

With regard to the inorganic alkoxide, the inorganic species is preferably not toxic in reconstructive medical applications, and can be advantageously selected from the group consisting of Ti, Zr, Al, Mg, Si, Zn, Mo, Nb, Ta, Sn, W, and V. Preferably the inorganic species is Al, Ti, Zr, Si, Mg or Zn. More preferably the inorganic species is Al, Si, Ti or Zr. The inorganic species can be Al. The inorganic species can be Ti. The inorganic species can be Zr. The inorganic species can be Mg. The inorganic species can be Si. The inorganic species can be Zn. The inorganic species can be Mo. The inorganic species can be Nb. The inorganic species can be Ta. The inorganic species can be Sn. The inorganic species can be W. The inorganic species can be V. Preferred alkoxides are selected from the group consisting of methoxide, ethoxide, propoxide, iso-propoxide, butoxide, iso-butoxide, sec-butoxide, and tert-butoxide.

One aspect of the invention is directed to a construct comprising a) a surface activated to the chemical bonding of an inorganic adhesion layer that provides for further attachment of moieties that modify the overall surface properties, where the surface contains no accessible, sufficiently reactive functional groups on the surface, where activation comprises generation of reactive functional groups on the surface; and b) an inorganic adhesion layer chemically bonded to the activated surface via the reactive functional groups; where the functional groups are reactive with an inorganic alkoxide to form the inorganic adhesion layer. The surface of such a construct comprises a polymer. The polymer can be selected from the group consisting of polyalkanes, polysiloxanes, polyalkylarenes, polyolefins, polythiols and polyphosphines. The surface functional groups of the construct are selected from the group consisting of oxy, oxo, carbonyl, carboxylic acid, and carboxylate groups.

The surface functional groups of the construct can be produced by chemical oxidation, using oxidizing agents such as permanganate, chlorite, chromic acid, chromate, osmium tetroxide, ruthenium tetroxide, iodate, peracids, peroxides, Fenton's reagent, lead tetraacetate, lead tetraacetate/Mn(II), ozone, or oxygen. Alternatively the surface functional groups can be generated using oxygen plasma discharge, or corona discharge.

The construct further comprises a self-assembled monolayer (SAM) bonded to the inorganic adhesion layer, where the SAM is selected from organic compounds comprising a phosphonic, carboxylic, sulfonic, phosphinic, phosphoric, sulfinic, or hydroxamic group. The SAM comprises a self-assembled monolayer of phosphonates (SAMP), where the phosphonates can be selected from the group consisting of hydrophobic phosphonates, cell-adhesive phosphonates and phosphonates capable of further metal-catalyzed coupling. The phosphonates can be selected from the group consisting of phosphonic acids of the structure 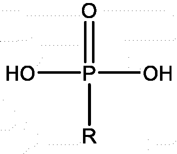 where the R group is selected from the group consisting of optionally substituted alkyl, optionally substituted heteroalkyl, optionally substituted alkenyl, optionally substituted heteroalkenyl, optionally substituted alkynyl, optionally substituted heteroalkynyl, optionally substituted aryl, optionally substituted arylalkyl, optionally substituted heteroaryl, and optionally substituted heteroarylalkyl, where heteroalkyl, heteroalkenyl, heteroalkynyl, heteroaryl and heteroarylalkyl contain one or more heteroatoms selected from the group consisting of O, N and S. The hydrophobic phosphonates of the construct can be selected from the group consisting of R = C₃-C₃₀ alkyl, and the cell-adhesive phosphonates are selected from the group consisting of R = C₃-C₃₀ alkyl substituted with a further phosphonate group. The cell-adhesive phosphonates can be selected from the group consisting of C₃-C₃₀ α,ω -diphosphonates.

The construct can have a SAMP that further comprises an anti-infective agent covalently bound thereto. Suitable anti-infective agents include an antimicrobial selected from the group consisting of amikacin, gentamicin, kanamycin, neomycin, netilmicin, tobramycin, paromomycin, geldanamycin, herbimycin, loracarbef, ertapenem, doripenem, imipenem/cilastatin, meropenem, cefadroxil, cefazolin, cefalotin, cefalexin, cefaclor, cefamandole, cefoxitin, cefprozil, cefuroxime, cefditoren, cefoperazone, cefotaxime, cefpodoxime, ceftazidime, ceftibuten, ceftizoxime, ceftriaxone, cefepime, ceftaroline fosamil, ceftobiprole, teicoplanin, vancomycin, telavancin, clindamycin, lincomycin, daptomycin, azithromycin, clarithromycin, dirithromycin, erythromycin, roxithromycin, troleandomycin, telithromycin, spectinomycin, spiramycin, aztreonam, furazolidone, nitrofurantoin, amoxicillin, ampicillin, azlocillin, carbenicillin, cloxacillin, dicloxacillin, flucloxacillin, mezlocillin, methicillin, nafcillin, oxacillin, penicillin G, penicillin V, piperacillin, temocillin, ticarcillin, amoxicillin/clavulanate, ampicillin/sulbactam, piperacillin/tazobactam, ticarcillin/clavulanate, bacitracin, colistin, polymyxin b, ciprofloxacin, enoxacin, gatifloxacin, levofloxacin, lomefloxacin, moxifloxacin, nalidixic acid, norfloxacin, ofloxacin, trovafloxacin, grepafloxacin, sparfloxacin, temafloxacin, mafenide, sulfonamidochrysoidine, sulfacetamide, sulfadiazine, silver, sulfadiazine, sulfamethizole, sulfamethoxazole, sulfanilimide, sulfasalazine, sulfisoxazole, trimethoprim, trimethoprim-sulfamethoxazole, demeclocycline, doxycycline, minocycline, oxytetracycline, tetracycline, clofazimine, dapsone, capreomycin, cycloserine, ethambutol, ethionamide, isoniazid, pyrazinamide, rifampicin, rifabutin, rifapentine, streptomycin, arsphenamine, chloramphenicol, fosfomycin, fusidic acid, linezolid, metronidazole, mupirocin, platensimycin, quinupristin/dalfopristin, rifaximin, thiamphenicol, tigecycline, tinidazole, pharmaceutically acceptable salts thereof, and mixtures of two or more thereof. Further, the anti-infective agent can be selected from the group consisting of chlorhexidine, biguanides, cationic ammonium compounds, pharmaceutically acceptable salts thereof, and mixtures of two or more thereof. The anti-infective agent can also be selected from the group consisting of cationic ammonium compounds, cationic ammonium dendrimers, silver, copper, cationic species and mixtures of two or more thereof. The cationic ammonium compounds can be selected from the group consisting of choline and choline derivatives. Alternatively, the anti-infective agent can comprise polysaccharides, chitosan, partially acetylated chitosan, polyglucosamine, chitosan diols, and other polyols (such as polyvinylalcohol) and aminoalcohols.

Another aspect of the invention is directed to a method of forming an inventive construct comprising the steps of a) activating a surface to chemical bonding of an inorganic adhesion layer that provides for further attachment of moieties that modify the overall surface properties, wherein the surface contains no accessible, sufficiently reactive functional groups on the surface, the activating comprising producing reactive functional groups on the surface to provide an activated surface; and b) chemically bonding an inorganic adhesion layer to the functional groups of the activated surface; where the functional groups are reactive with an inorganic alkoxide to form the inorganic adhesion layer. The surface of the method comprises a polymer, which can be selected from the group consisting of polyalkanes, polysiloxanes, polyalkylarenes, polyolefins, polythiols and polyphosphines. The surface functional groups of the method are selected from the group consisting of oxy, oxo, carbonyl, carboxylic acid, and carboxylate groups. The activating step of the method can comprise chemical oxidation, using such chemical oxidizing agents as permanganate, chlorite, chromic acid, chromate, osmium tetroxide, ruthenium tetroxide, iodate, peracids, peroxides, Fenton's reagent, lead tetraacetate, lead tetraacetate/Mn(II), ozone, of oxygen. Alternatively the activating step of the method can comprise oxygen plasma discharge, or corona discharge.

Another aspect of the invention is directed to a method of activating and coating an unactivated surface with an inorganic oxide adhesion layer, comprising the steps of a) activating the surface of an unactivated substrate to the chemical bonding of an inorganic adhesion layer; b) providing a coating mixture comprising an organic solvent containing a reactive inorganic compound that is dissolved and/or dispersed in the solvent; and c) suspending the activated substrate in the coating mixture for a time and temperature sufficient to form an inorganic oxide coating on the activated surface, to provide a surface coated with an inorganic oxide adhesion layer; where the inorganic compound is selected from the group consisting of the alkoxides of Ti, Zr, Al, Mg, Si, Zn, Mo, Nb, Ta, Sn, W, and V. The method can further comprise d) removing the coated substrate from the coating solution; e) rinsing with a solvent to provide a rinsed coated substrate; and f) heating the rinsed coated substrate to 35 to 40°C. Alternatively, steps b) and c) can be replaced by vapor deposition of an inorganic alkoxide onto the surface, providing the inorganic oxide adhesion layer.

Another aspect of the invention is directed to a construct having a SAMP, as disclosed above, where the R group is dodecyl-9-ynyl, further coupled at the alkyne using a metal-catalyzed reaction. The dodecyl-9-ynyl R group can also be further coupled at the alkyne using a click reaction.

Alternatively, not according to the invention, an aspect is directed to an adhesion layer-coated construct without a SAM or SAMP, further reacted directly at the inorganic adhesion layer with an organic moiety selected from the group consisting of electrochemically active moieties, photochemically active moieties, cell-attractive moieties, cell-adhesive moieties and anti-infective moieties. The anti-infective agent can comprise polysaccharides, chitosan, partially acetylated chitosan, polyglucosamine, chitosan diols, and other polyols (such as polyvinylalcohol) and aminoalcohols. Preferably the anti-infective moieties are selected from the group consisting of polysaccharides, chitosan, partially acetylated chitosan, polyglucosamine, chitosan diols, polyols, aminoalcohols and mixtures of two or more thereof. Proteins can also be directly attached to the inorganic adhesion layer. Suitable proteins include heparin and heparin derivatives such as heparin-functionalized phosphonate and silane pre-molecule (Heparin PUL and Heparin Silane, respectively).

Thus, one aspect not according to the invention is directed to stainless steel (SS) coupons which were oxygen plasma-oxidized on both sides and dipped into a solution of titanium (IV) butoxide to form a Ti(IV) butoxide adhesion layer covalently attached to the SS surface. This coated SS surface was functionalized with heparin using either heparin-functionalized phosphonate or silane pre-molecule, and the heparin was found to be in its native form (vs. denatured) by XPS elemental analysis (see Example 5).

One aspect of the invention is directed to a construct comprising: a) an activated surface comprising chemically accessible, reactive functional groups; and b) an inorganic alkoxide adhesion layer chemically bonded to the reactive functional groups of the activated surface; where the reactive functional groups are reactive with an inorganic alkoxide to form the inorganic adhesion layer, and the inorganic adhesion layer provides additional functional groups for further attachment of moieties that modify the overall surface properties. The activated surface of the construct comprises an intrinsically unreactive surface towards chemical bonding of an inorganic adhesion layer, which has been treated to produce chemically accessible, reactive functional groups on the surface thereby providing activation. The surface of the construct comprises a polymer that is intrinsically unreactive towards chemical bonding of an inorganic adhesion layer. The polymer can be selected from the group consisting of polyalkanes, polysiloxanes, polyalkylarenes, polyolefins, polythiols and polyphosphines.

The surface reactive functional groups are selected from the group consisting of oxy, oxo, carbonyl, carboxylic acid, and carboxylate groups. The surface reactive functional groups can be produced by chemical oxidation. The chemical oxidation can comprise treatment with an oxidizing agent selected from the group consisting of permanganate, chlorite, chromic acid, chromate, osmium tetroxide, ruthenium tetroxide, iodate, peracids, peroxides, Fenton's reagent, lead tetraacetate, lead tetraacetate/Mn(II), ozone, and oxygen.

Alternatively the surface reactive functional groups can be produced by oxygen plasma discharge, or corona discharge.

The inorganic adhesion layer of the construct can comprise an inorganic oxide selected from the group consisting of the oxides of Ti, Zr, Al, Mg, Si, Zn, Mo, Nb, Ta, Sn, W, V, and mixtures of two or more thereof. Preferably the inorganic oxide adhesion layer is selected from the group consisting of the oxides of Al, Ti, Zr, Si, Mg, Zn, and mixtures of two or more thereof.

The construct further comprises a self-assembled monolayer (SAM) bonded to the additional functional groups of the inorganic adhesion layer, where the SAM is selected from organic compounds comprising a phosphonic, carboxylic, sulfonic, phosphinic, phosphoric, sulfinic, or hydroxamic group. The SAM comprises a self-assembled monolayer of phosphonates (SAMP). The phosphonates can be selected from the group consisting of hydrophobic phosphonates and cell-adhesive phosphonates. The hydrophobic and cell-adhesive phosphonates can be selected from the group consisting of phosphonic acids of structure 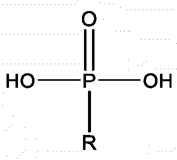 where the R group is selected from the group consisting of optionally substituted alkyl, optionally substituted heteroalkyl, optionally substituted alkenyl, optionally substituted heteroalkenyl, optionally substituted alkynyl, optionally substituted heteroalkynyl, optionally substituted aryl, optionally substituted arylalkyl, optionally substituted heteroaryl, and optionally substituted heteroarylalkyl, where heteroalkyl, heteroalkenyl, heteroalkynyl, heteroaryl and heteroarylalkyl contain one or more heteroatoms selected from the group consisting of O, N and S.

The R group of the construct can be dodecyl-9-ynyl, further coupled at the alkyne functional group using a metal-catalyzed reaction. The R group of the construct can be dodecyl-9-ynyl, further coupled at the alkyne functional group using a click reaction.

The hydrophobic phosphonates can be selected from the group consisting of R = C₃-C₃₀ alkyl, and the cell-adhesive phosphonates are selected from the group consisting of R = C₃-C₃₀ alkyl substituted with a further phosphonate group. The cell-adhesive phosphonates can be selected from the group consisting of C₃-C₃₀ α,ω)-diphosphonates.

The SAMP of the construct can further comprise an anti-infective agent or an antithrombogenic agent covalently bound thereto. The anti-infective agent can be an antimicrobial selected from the group consisting of amikacin, gentamicin, kanamycin, neomycin, netilmicin, tobramycin, paromomycin, geldanamycin, herbimycin, loracarbef, ertapenem, doripenem, imipenem/cilastatin, meropenem, cefadroxil, cefazolin, cefalotin, cefalexin, cefaclor, cefamandole, cefoxitin, cefprozil, cefuroxime, cefditoren, cefoperazone, cefotaxime, cefpodoxime, ceftazidime, ceftibuten, ceftizoxime, ceftriaxone, cefepime, ceftaroline fosamil, ceftobiprole, teicoplanin, vancomycin, telavancin, clindamycin, lincomycin, daptomycin, azithromycin, clarithromycin, dirithromycin, erythromycin, roxithromycin, troleandomycin, telithromycin, spectinomycin, spiramycin, aztreonam, furazolidone, nitrofurantoin, amoxicillin, ampicillin, azlocillin, carbenicillin, cloxacillin, dicloxacillin, flucloxacillin, mezlocillin, methicillin, nafcillin, oxacillin, penicillin G, penicillin V, piperacillin, temocillin, ticarcillin, amoxicillin/clavulanate, ampicillin/sulbactam, piperacillin/tazobactam, ticarcillin/clavulanate, bacitracin, colistin, polymyxin b, ciprofloxacin, enoxacin, gatifloxacin, levofloxacin, lomefloxacin, moxifloxacin, nalidixic acid, norfloxacin, ofloxacin, trovafloxacin, grepafloxacin, sparfloxacin, temafloxacin, mafenide, sulfonamidochrysoidine, sulfacetamide, sulfadiazine, silver, sulfadiazine, sulfamethizole, sulfamethoxazole, sulfanilimide, sulfasalazine, sulfisoxazole, trimethoprim, trimethoprim-sulfamethoxazole, demeclocycline, doxycycline, minocycline, oxytetracycline, tetracycline, clofazimine, dapsone, capreomycin, cycloserine, ethambutol, ethionamide, isoniazid, pyrazinamide, rifampicin, rifabutin, rifapentine, streptomycin, arsphenamine, chloramphenicol, fosfomycin, fusidic acid, linezolid, metronidazole, mupirocin, platensimycin, quinupristin/dalfopristin, rifaximin, thiamphenicol, tigecycline, tinidazole, pharmaceutically acceptable salts thereof, and mixtures of two or more thereof.

Alternatively, the anti-infective agent can be selected from the group consisting of polysaccharides, chitosan, partially acetylated chitosan, polyglucosamine, chitosan diols, polyols, aminoalcohols and mixtures of two or more thereof.

The anti-infective agent can be selected from the group consisting of chlorhexidine, biguanides, cationic ammonium compounds, cationic ammonium dendrimers, silver, copper, cationic species and mixtures of two or more thereof. The cationic ammonium compounds can be selected from the group consisting of choline and choline derivatives.

The antithrombogenic agent can be heparin.

The construct of the invention can further comprise cells attached to the coated surface of the construct, where the cells are selected from the group consisting of fibroblasts, endothelial cells, keratinocytes, osteoblasts, chondroblasts, chondrocytes, hepatocytes, macrophages, cardiac muscle cells, smooth muscle cells, skeletal muscle cells, tendon cells, ligament cells, epithelial cells, stem cells, neural cells, PC12 cells, neural support cells, Schwann cells, radial glial cells, cells that form neurospheres, neural tumor cells, glioblastoma cells and neuroblastoma cells. The fibroblasts can comprise NIH 3T3 fibroblasts.

The construct can further comprise an extracellular matrix (ECM). The construct can also be decelluarized to leave the ECM.

In another aspect not according to the invention, the construct as described above can have the inorganic adhesion layer directly attached to an organic moiety selected from the group consisting of electrochemically active moieties, photochemically active moieties, cell-attractive moieties, cell-adhesive moieties and anti-infective moieties, without an intervening SAM or SAMP layer. The anti-infective moieties can be selected from the group consisting of polysaccharides, chitosan, partially acetylated chitosan, polyglucosamine, chitosan diols, polyols, aminoalcohols and mixtures of two or more thereof.

Another aspect of the invention is directed to a method of forming a construct as described above, the method comprising the steps of a) providing a surface that is intrinsically unreactive towards chemical bonding of an inorganic adhesion layer; b) activating the intrinsically unreactive surface to chemical bonding of an inorganic adhesion layer by treating the unreactive surface to produce reactive functional groups on the surface thereby providing an activated surface; and c) chemically bonding an inorganic adhesion layer to the reactive functional groups of the activated surface; where the reactive functional groups are reactive with an inorganic alkoxide to form the inorganic adhesion layer; and where the inorganic adhesion layer provides additional functional groups for further attachment of moieties that modify the overall surface properties.

**In** the above method, the surface comprises a polymer that is intrinsically unreactive towards chemical bonding of an inorganic adhesion layer. The polymer can be selected from the group consisting of polyalkanes, polysiloxanes, polyalkylarenes, polyolefins, polythiols and polyphosphines. The surface functional groups generated by activation are selected from the group consisting of oxy, oxo, carbonyl, carboxylic acid, and carboxylate groups. The activating step can comprise chemical oxidation. The chemical oxidation can comprise treatment with an oxidizing agent selected from the group consisting of permanganate, chlorite, chromic acid, chromate, osmium tetroxide, ruthenium tetroxide, iodate, peracids, peroxides, Fenton's reagent, lead tetraacetate, lead tetraacetate/Mn(II), ozone, and oxygen.

Alternatively the activating step can comprise oxygen plasma discharge, or corona discharge.

The inorganic adhesion layer of the method can comprise an inorganic oxide selected from the group consisting of the oxides of Ti, Zr, Al, Mg, Si, Zn, Mo, Nb, Ta, Sn, W, V, and mixtures of two or more thereof. Preferably the inorganic oxide adhesion layer is selected from the group consisting of the oxides of Al, Ti, Zr, Si, Mg, Zn, and mixtures of two or more thereof.

Another aspect of the invention is directed to a method of activating an unactivated substrate surface and coating with an inorganic oxide adhesion layer, comprising the steps of: a) activating the surface of an unactivated substrate to chemical bonding of an inorganic adhesion layer by producing reactive functional groups on the surface to form an activated substrate; b) providing a coating mixture comprising an organic solvent containing a reactive inorganic compound that is dissolved and/or dispersed in the solvent; and c) suspending the activated substrate in the coating mixture for a time and at a temperature sufficient for the reactive functional groups and the inorganic compound to react and form an inorganic oxide coating on the activated surface of the substrate, providing a substrate coated with an inorganic oxide adhesion layer; where the inorganic compound is selected from the group consisting of the alkoxides of Ti, Zr, Al, Mg, Si, Zn, Mo, Nb, Ta, Sn, W, and V.

The activating step of the method can comprise chemical oxidation. Chemical oxidation can comprise treatment with an oxidizing agent selected from the group consisting of permanganate, chlorite, chromic acid, chromate, osmium tetroxide, ruthenium tetroxide, iodate, peracids, peroxides, Fenton's reagent, lead tetraacetate, lead tetraacetate/Mn(II), ozone, and oxygen.

Steps b) and c) of the method can be replaced by vapor deposition of an inorganic alkoxide onto the surface of the activated substrate and reaction of the reactive functional groups with the inorganic alkoxide to form an inorganic oxide adhesion layer bonded thereto.

Alternatively the activating step can comprise oxygen plasma discharge, or corona discharge.

The method can further comprise: d) removing the coated substrate from the coating solution; e) rinsing with a solvent to provide a rinsed coated substrate; and f) heating the rinsed coated substrate to 35 to 40°C.

**In** summary, the present method converts an otherwise unreactive material into one that is reactive at the surface. This surface reactivity can be controlled by the activation method and by nature of the coating layer of the activated surface, so that cell attachment, spreading and ECM formation are possible. Such means to direct the surface properties of materials provide many possible applications in medicine and other biomedical and biological fields.

### EXAMPLES

*General.* All materials were procured from commercial sources. Solvents and chemical reagents include methanol (Sigma Aldrich), 2-propanol (Sigma Aldrich), tert-butanol (Fisher Scientific), 200 proof ethanol (Pharmco-Aaper), xylene (EMD Millipore Corporation), toluene (EMD Chemical Inc.), hexanes (Sigma Aldrich), titanium(IV) iso-propoxide (Sigma Aldrich), 1,4-butanediphosphonic acid (Acros Organics), 1,12-dodecanediylbis(phosphonic acid) (Sigma Aldrich), and octadecylphosphonic acid (Alfa Aesar, Sigma Aldrich).

### Example 1. Oxygen Plasma-Oxidized PDMS having a titanium iso-propoxide adhesion layer with an ODPA self-assembled monolayer

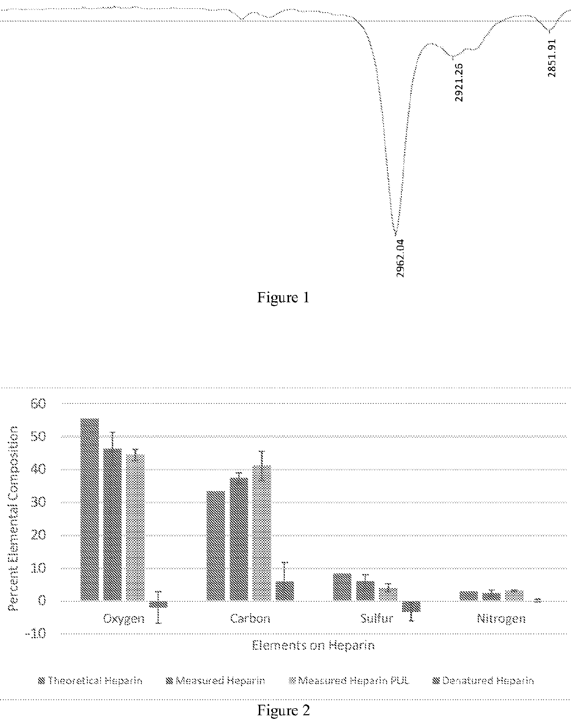

Polydimethylsiloxane (PDMS) coupons were oxygen plasma-oxidized on one side and were dipped into a solution of titanium iso-propoxide in toluene at a concentration of 10µL/mL. The samples were left submerged in the solution for 15 minutes. The coupons were removed from solution, and it was noted that the surfaces of these PDMS coupons had become slightly less translucent. The coupons were heated at 35 °C for 1 min, rinsed with ethanol, and then placed in a solution of octadecylphosphonic acid (ODPA) in toluene at a concentration of 0.5 mg/mL. The coupons were kept in this solution for several hours, removed from solution, heated at 35 °C for 1 min, and rinsed with ethanol. The appearance of the coupon surfaces did not change during this procedure. Infrared spectral analysis showed the presence of a monolayer of octadecylphosphonate on the PDMS surface (Figure 1). The large peak is attributable to the PDMS methyl groups; the peaks at 2921 and 2851 cm⁻¹ are characteristic of octadecylphosphonate (ODPA) self-assembled monolayers. In contrast, a control PDMS coupon that was not pretreated with titanium iso-propoxide to form an adhesion layer, showed no evidence of a phosphonate SAM after analogous ODPA treatment. Similar results were observed using 11-hydroxyundecylphosphonic acid. Infrared Spectroscopy. Infrared (IR) spectroscopy enables the detection of functional groups in a molecule by identifying unique peaks corresponding to the stretching and bending of chemical bonds. This same technique can be applied to SAMs on both optically transparent (transmission mode) and reflective (grazing angle spectral reflectance mode) substrates. IR can evaluate successful monolayer preparation and monitor degradation, as well as determine the degree of ordering in a SAM surface. Antisymmetric and symmetric methylene stretches are diagnostic peaks for alkyl-based monolayers, and appear in the vicinity of 2920 and 2850 cm⁻¹ respectively. The wavenumbers for methylene group stretching modes are understood to be diagnostic of whether the chains exist in an *all-trans* configuration ("ordered" or crystalline state) or in a random configuration ("disordered" and "liquid-like" film). A well-ordered film in this work is defined to be characterized by antisymmetric methylene stretching wavenumber below 2920 cm⁻¹ and symmetric methylene stretching wavenumber below 2850 cm⁻¹. To assess film quality, ATR-FTIR data were taken using a Nicolet TMiSTM50 **FT-IR** Spectrometer.

Example 2. A coupon of treated PDMS is prepared as described in Example 1, and is treated with a solution of zirconium n-butoxide dissolved in toluene at a concentration of 0.5 mg/mL. The coupon is heated at 35 °C for 1 min, rinsed with ethanol, and is then placed in a solution of phosphonodec-9-yne in toluene at a concentration of 0.5 mg/mL. The coupon is kept in this solution for several hours, removed from solution, heated at 35 °C for 1 min, rinsed with ethanol, and analyzed by **IR** spectroscopy for the peaks that are characteristic of a terminal alkynyl group at ca. 2135 and 3325 cm⁻¹. A copper/palladium-catalyzed coupling reaction is then carried out with bromobenzene as an example to yield the 10-phenyl-coupled product.

Example 3. A coupon of treated PDMS is prepared as described in Example 1, and is treated with a solution of zirconium n-butoxide dissolved in toluene at a concentration of 0.5 mg/mL. The coupon is heated at 35 °C for 1 min, rinsed with ethanol, and is then placed in a solution of phosphonodec-9-yne in toluene at a concentration of 0.5 mg/mL. The coupon is kept in this solution for several hours, removed from solution, heated at 35 °C for 1 min, rinsed with ethanol, and analyzed by **IR** spectroscopy for the peaks that are characteristic of a terminal alkynyl group at ca. 2135 and 3325 cm⁻¹. A copper-catalyzed "click" reaction is then performed using phenyl azide to give the phenyltriazole-terminated phosphonate.

Example 4. A coupon of treated PDMS is prepared as described in Example 1, and is treated with a solution of zirconium n-butoxide dissolved in toluene at a concentration of 0.5 mg/mL. The coupon is heated at 35 °C for 1 min, rinsed with ethanol, and is then placed in a solution of glycerol in ethanol at a concentration of 0.5 mg/mL. The coupon is kept in this solution for several hours, removed from solution, heated at 35 °C for 1 min, rinsed with ethanol, and analyzed by **IR** spectroscopy for the peaks that are characteristic of hydroxyl, ether and aliphatic groups at around 1050, 2980 and 3100 cm⁻¹.

Example 5. Covalently-Bound Heparin on Ti-Functionalized Stainless Steel *General.* All materials were procured from commercial sources. Solvents and chemical reagents include anhydrous toluene (Sigma Aldrich), tert-butanol (Fisher Scientific), 200 proof ethanol (Acros Organics), reagent alcohol (Fisher Scientific), titanium(IV) butoxide (Sigma Aldrich), monoamine functionalized trialkoxy silane and phosphonate (Gelest, Sikemia), Heparin sodium salt (Sigma Aldrich).

Stainless Steel (SS) coupons were oxygen plasma-oxidized on both sides and were dipped into a solution of titanium (IV) butoxide in toluene at a concentration of 3% (v/v). The samples were left submerged in the solution for 15 minutes with constant stirring at 350 rpm. The Ti(IV) butoxide treated coupons were taken out of solution and allowed to dry inside a chemical hood for 5 minutes. The samples were then placed in an oven at 130°C for 10 min, then sonicated with reagent alcohol for 10 min (twice), and vacuum dried for 10 minutes. The coupons at this point had visible shades of gray on the surface when compared to control SS coupons. The samples were placed in a 1.5 - 15 mM ethanolic solution of heparin-functionalized phosphonate or silane pre-molecule (Heparin PUL or Heparin Silane) and were kept in this solution at a temperature ranging from 24 - 37°C overnight. The coupons were rinsed and sonicated in reagent alcohol for 10 minutes (twice) and vacuum dried for 10 minutes. The appearance of the coupon surfaces did not change during this procedure. X-ray photon spectroscopy analysis showed an elemental composition pattern consistent with the native form of the heparin molecule on the stainless-steel surface (Figure 2). Heparin is a sugar dimer that repeats many times to make up a distribution of different molecular weights. The dimer is composed of five chemical elements. Four of those elements (carbon, oxygen, sulfur, and nitrogen) can be detected by XPS. **In** addition, the theoretical percent composition of these elements in heparin can be calculated and correlated with the experimentally observed elemental percent composition values as determined by XPS. We found by XPS analysis on native and denatured heparin, using noncovalently-bound native heparin, negative control (heat denatured covalently bound heparin, temperature > 100°C), and covalently bound heparin-functionalized phosphonate on stainless steel, that the elemental percent composition pattern of noncovalently-bound native heparin and covalently bound heparin-functionalized phosphonate are comparable to the theoretical percent elemental composition of the heparin dimer. The biggest change in heparin percent elemental composition between the heat denatured covalently bound heparin group and covalently bound heparin-functionalized phosphonate was the significant loss of elemental percent carbon, oxygen, sulfur, and nitrogen.

X-ray Photon Spectroscopy. X-ray Photon Spectroscopy (XPS) is technique that measures the percent elemental composition of a surface-bound molecule by identifying the presence of specific elements within known functional groups in a molecule within a 10-nm depth profile. This same technique can be applied to surface-bound molecules to determine the presence of elements within functional regions, in addition to measuring deviations from native elemental percent composition values. In the present case, XPS can evaluate successful surface preparation and monitor degradation of important domains in heparin due to different strategies used to bind heparin to stainless steel. Theoretically, the repeating dimeric unit in heparin has an elemental percent composition pattern of carbon (33%), oxygen (55%), sulfur (8.0%), and nitrogen (2.8%). The native elemental composition of heparin supports the interaction of heparin with anti-thrombin due to the key-lock interaction leading to structural changes on anti-thrombin and its activity to prevent thrombus formation. Significant changes in the elemental percent composition of sulfur are understood to be diagnostic of whether heparin is in its native or denatured form at the surface.

The form of the covalently-bound heparin as prepared above was shown to be native by comparing its elemental percent composition versus the experimentally measured elemental percent composition values of native heparin; there were no statistically significant differences, thereby confirming the native covalently-bound form.

### OTHER EMBODIMENTS

From the above description, one skilled in the art can easily ascertain the essential characteristics of the present invention, and can make various changes and modifications of the invention to adapt it to various usages and conditions. Thus, other embodiments are also within the scope of the present claims.

## Claims

1. A construct for cell growth, reconstructive medicine or medical devices comprising:
a) an oxidized polymer surface comprising chemically accessible, reactive oxy, oxo, carbonyl, carboxylic acid, or carboxylate functional groups reactive with inorganic alkoxides; and
b) an inorganic oxide adhesion layer chemically bonded to said reactive functional groups of said polymer surface, said inorganic oxide adhesion layer comprising surface alkoxide groups; and
c) a self-assembled phosphonate monolayer (SAMP) bonded to the surface alkoxide groups of said inorganic oxide adhesion layer,
wherein the polymer surface, prior to oxidation, does not have exposed surface functional groups in sufficient density or reactivity to form covalent or coordinative bonds with inorganic alkoxides.

2. The construct of claim 1, wherein said polymer surface comprises a polymer selected from the group consisting of polyalkanes, polysiloxanes, polyalkylarenes, polyolefins, polythiols and polyphosphines.

3. The construct of claim 1 or claim 2, wherein said inorganic oxide adhesion layer comprises an inorganic oxide selected from the group consisting of the oxides of Ti, Zr, Al, Mg, Si, Zn, Mo, Nb, Ta, Sn, W, V, and mixtures of two or more thereof.

4. The construct of any one of claims 1 to 3, wherein said phosphonates are selected from the group consisting of hydrophobic phosphonates and cell-adhesive phosphonates, optionally wherein said hydrophobic and cell-adhesive phosphonates are selected from the group consisting of phosphonic acids of structure 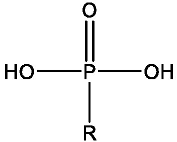 wherein the R group is selected from the group consisting of optionally substituted alkyl, optionally substituted heteroalkyl, optionally substituted alkenyl, optionally substituted heteroalkenyl, optionally substituted alkynyl, optionally substituted heteroalkynyl, optionally substituted aryl, optionally substituted arylalkyl, optionally substituted heteroaryl, and optionally substituted heteroarylalkyl, where heteroalkyl, heteroalkenyl, heteroalkynyl, heteroaryl and heteroarylalkyl contain one or more heteroatoms selected from the group consisting of O, N and S, optionally wherein said hydrophobic phosphonates are selected from the group consisting of R = C₃-C₃₀ alkyl, and said cell-adhesive phosphonates are selected from the group consisting of R = C₃-C₃₀ alkyl substituted with a further phosphonate group, optionally wherein said cell-adhesive phosphonates are selected from the group consisting of C₃-C₃₀ α,ω-diphosphonates, optionally wherein said R group is dodecyl-9-ynyl, further coupled at the alkyne functional group using a metal catalyzed reaction, or optionally where said R group is dodecyl-9-ynyl, further coupled at the alkyne functional group using a click reaction.

5. The construct of any one of claims 1 to 4, wherein said SAMP further comprises an anti-infective agent or an antithrombogenic agent, for example heparin, covalently bound thereto, optionally wherein said anti-infective agent is an antimicrobial selected from the group consisting of amikacin, gentamicin, kanamycin, neomycin, netilmicin, tobramycin, paromomycin, geldanamycin, herbimycin, loracarbef, ertapenem, doripenem, imipenem/cilastatin, meropenem, cefadroxil, cefazolin, cefalotin, cefalexin, cefaclor, cefamandole, cefoxitin, cefprozil, cefuroxime, cefditoren, cefoperazone, cefotaxime, cefpodoxime, ceftazidime, ceftibuten, ceftizoxime, ceftriaxone, cefepime, ceftaroline fosamil, ceftobiprole, teicoplanin, vancomycin, telavancin, clindamycin, lincomycin, daptomycin, azithromycin, clarithromycin, dirithromycin, erythromycin, roxithromycin, troleandomycin, telithromycin, spectinomycin, spiramycin, aztreonam, furazolidone, nitrofurantoin, amoxicillin, ampicillin, azlocillin, carbenicillin, cloxacillin, dicloxacillin, flucloxacillin, mezlocillin, methicillin, nafcillin, oxacillin, penicillin G, penicillin V, piperacillin, temocillin, ticarcillin, amoxicillin/clavulanate, ampicillin/sulbactam, piperacillin/tazobactam, ticarcillin/clavulanate, bacitracin, colistin, polymyxin b, ciprofloxacin, enoxacin, gatifloxacin, levofloxacin, lomefloxacin, moxifloxacin, nalidixic acid, norfloxacin, ofloxacin, trovafloxacin, grepafloxacin, sparfloxacin, temafloxacin, mafenide, sulfonamidochrysoidine, sulfacetamide, sulfadiazine, silver, sulfadiazine, sulfamethizole, sulfamethoxazole, sulfanilimide, sulfasalazine, sulfisoxazole, trimethoprim, trimethoprim-sulfamethoxazole, demeclocycline, doxycycline, minocycline, oxytetracycline, tetracycline, clofazimine, dapsone, capreomycin, cycloserine, ethambutol, ethionamide, isoniazid, pyrazinamide, rifampicin, rifabutin, rifapentine, streptomycin, arsphenamine, chloramphenicol, fosfomycin, fusidic acid, linezolid, metronidazole, mupirocin, platensimycin, quinupristin/dalfopristin, rifaximin, thiamphenicol, tigecycline, tinidazole, pharmaceutically acceptable salts thereof, and mixtures of two or more thereof, or optionally wherein said anti-infective agent is selected from the group consisting of polysaccharides, chitosan, partially acetylated chitosan, polyglucosamine, chitosan diols, polyols, aminoalcohols and mixtures of two or more thereof, or optionally wherein said anti-infective agent is selected from the group consisting of chlorhexidine, biguanides, cationic ammonium compounds, cationic ammonium dendrimers, silver, copper, cationic species and mixtures of two or more thereof, optionally wherein said cationic ammonium compounds are selected from the group consisting of choline and choline derivatives.

6. The construct of any one of claims 1 to 5, further comprising cells attached to the coated polymer surface of said construct, wherein said cells are selected from the group consisting of fibroblasts, endothelial cells, keratinocytes, osteoblasts, chondroblasts, chondrocytes, hepatocytes, macrophages, cardiac muscle cells, smooth muscle cells, skeletal muscle cells, tendon cells, ligament cells, epithelial cells, stem cells, neural cells, PC12 cells, neural support cells, Schwann cells, radial glial cells, cells that form neurospheres, neural tumor cells, glioblastoma cells and neuroblastoma cells, optionally wherein said fibroblasts comprise NIH 3T3 fibroblasts.

7. The construct of claim 6, further comprising an extracellular matrix (ECM) secreted by cells and providing structural and biochemical support thereto.

8. The construct of claim 7, which is decelluarized to leave the ECM secreted by the cells attached to the construct.

9. A method of forming the construct of any preceding claim, comprising:
a) providing a polymer surface that is intrinsically unreactive towards chemical bonding of an inorganic adhesion layer;
b) oxidizing the polymer surface to form chemically accessible, reactive oxy, oxo, carbonyl, carboxylic acid, or carboxylate functional groups, thereby providing an activated surface;
c) chemically bonding an inorganic oxide adhesion layer with surface alkoxide groups to said reactive functional groups of said activated polymer surface; and
d) bonding a self-assembled phosphonate monolayer (SAMP) to the surface alkoxide groups of said inorganic oxide adhesion layer.

10. A method of activating a polymer substrate surface and coating the said activated substrate polymer surface with an inorganic oxide adhesion layer, comprising the steps of:
a) activating the polymer surface of an unactivated substrate to chemical bonding of an inorganic adhesion layer by oxidizing said polymer surface to form reactive oxy, oxo, carbonyl, carboxylic acid, or carboxylate functional groups on said polymer surface and thereby form an activated substrate;
b) providing a coating mixture comprising an organic solvent containing a reactive inorganic alkoxide compound that is dissolved and/or dispersed in the solvent;
c) suspending said activated polymer substrate in said coating mixture for a time and at a temperature sufficient to react said reactive functional groups with said inorganic alkoxide compound and form an inorganic oxide coating on the activated surface of said polymer substrate, to provide a substrate coated with an inorganic oxide adhesion layer;
d) optionally, removing the coated substrate from the coating solution;
e) optionally, rinsing with a solvent to provide a rinsed coated substrate; and
f) optionally, heating the rinsed coated substrate to 35 to 40°C;
g) bonding a self-assembled phosphonate monolayer (SAMP) to the surface alkoxide groups of said inorganic adhesion layer,
wherein said inorganic alkoxide compound is selected from the group consisting of the alkoxides of Ti, Zr, Al, Mg, Si, Zn, Mo, Nb, Ta, Sn, W, and V.

11. The method of claims 9 to 10, wherein said activating step comprises chemical oxidation, optionally wherein said chemical oxidation comprises treatment with an oxidizing agent selected from the group consisting of permanganate, chlorite, chromic acid, chromate, osmium tetroxide, ruthenium tetroxide, iodate, peracids, peroxides, Fenton's reagent, lead tetraacetate, lead tetraacetate/Mn(II), ozone, and oxygen, or wherein said activating step comprises oxygen plasma discharge, or corona discharge.

12. The method of any one of claims 10 to 11, wherein the coating steps are replaced by vapor depositing an inorganic alkoxide onto the surface of said activated substrate, and reacting said reactive functional groups with said inorganic alkoxide to form an inorganic oxide adhesion layer bonded thereto.

13. The construct of any of claims 1 to 4 or 6 to 8, wherein the SAMP is covalently bound to an organic moiety selected from the group consisting of electrochemically active moieties, photochemically active moieties, cell-attractive moieties, cell-adhesive moieties and anti-infective moieties.

## Patentansprüche

1. Konstrukt für Zellwachstum, rekonstruktive Medizin oder medizinische Vorrichtungen, umfassend:
a) eine oxidierte Polymeroberfläche, umfassend chemisch zugängliche, reaktive Oxy-, Oxo-, Carbonyl-, Carbonsäure- oder Carboxylat-Funktionsgruppen, die mit anorganischen Alkoxiden reaktiv sind; und
b) eine anorganische Oxid-Haftschicht, die chemisch an die reaktiven Funktionsgruppen der Polymeroberfläche gebunden ist, die anorganische Oxid-Haftschicht umfassend Oberflächenalkoxidgruppen; und
c) eine selbstorganisierte Phosphonat-Monoschicht (SAMP), die an die Oberflächenalkoxidgruppen der anorganischen Oxid-Haftschicht gebunden ist,
wobei die Polymeroberfläche vor der Oxidation keine freiliegenden Oberflächenfunktionsgruppen in einer ausreichenden Dichte oder mit einer ausreichenden Reaktivität aufweist, um kovalente oder koordinative Bindungen mit anorganischen Alkoxiden zu bilden.

2. Konstrukt nach Anspruch 1, wobei die Polymeroberfläche ein Polymer umfasst, das aus der Gruppe bestehend aus Polyalkanen, Polysiloxanen, Polyalkylarenen, Polyolefinen, Polythiolen und Polyphosphinen ausgewählt ist.

3. Konstrukt nach Anspruch 1 oder 2, wobei die anorganische Oxid-Haftschicht ein anorganisches Oxid umfasst, das aus der Gruppe bestehend aus den Oxiden von Ti, Zr, Al, Mg, Si, Zn, Mo, Nb, Ta, Sn, W, V und Mischungen aus zwei oder mehreren davon ausgewählt ist.

4. Konstrukt nach einem der Ansprüche 1 bis 3, wobei die Phosphonate aus der Gruppe bestehend aus hydrophoben Phosphonaten und zelladhäsiven Phosphonaten ausgewählt sind, wobei die hydrophoben und zelladhäsiven Phosphonate gegebenenfalls aus der Gruppe bestehend aus Phosphonsäuren der Struktur ausgewählt sind, 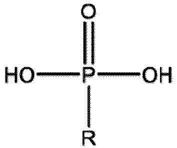 wobei die R-Gruppe aus der Gruppe bestehend aus gegebenenfalls substituiertem Alkyl, gegebenenfalls substituiertem Heteroalkyl, gegebenenfalls substituiertem Alkenyl, gegebenenfalls substituiertem Heteroalkenyl, gegebenenfalls substituiertem Alkinyl, gegebenenfalls substituiertem Heteroalkinyl, gegebenenfalls substituiertem Aryl, gegebenenfalls substituiertem Arylalkyl, gegebenenfalls substituiertem Heteroaryl und gegebenenfalls substituiertem Heteroarylalkyl ausgewählt ist, wobei Heteroalkyl, Heteroalkenyl, Heteroalkinyl, Heteroaryl und Heteroarylalkyl ein oder mehrere Heteroatome enthalten, die aus der Gruppe bestehend aus O, N und S ausgewählt sind, wobei gegebenenfalls die hydrophoben Phosphonate aus der Gruppe bestehend aus R = C₃-C₃₀-Alkyl ausgewählt sind und die zelladhäsiven Phosphonate aus der Gruppe bestehend aus R = C₃-C₃₀-Alkyl, das mit einer weiteren Phosphonatgruppe substituiert ist, ausgewählt sind, wobei die zelladhäsiven Phosphonate gegebenenfalls aus der Gruppe bestehend aus C₃-C₃₀-α,ω-Diphosphonaten ausgewählt sind, wobei die R-Gruppe gegebenenfalls Dodecyl-9-ynyl ist, das ferner an der Alkin-Funktionsgruppe unter Verwendung einer metallkatalysierten Reaktion gekoppelt ist, oder wobei die R-Gruppe gegebenenfalls Dodecyl-9-ynyl ist, das ferner an der Alkin-Funktionsgruppe unter Verwendung einer Click-Reaktion gekoppelt ist.

5. Konstrukt nach einem der Ansprüche 1 bis 4, wobei die SAMP ferner ein antiinfektiöses Mittel oder ein antithrombogenes Mittel, beispielsweise Heparin, umfasst, das kovalent daran gebunden ist, wobei das antiinfektiöse Mittel gegebenenfalls ein antimikrobielles Mittel ist, das aus der Gruppe bestehend aus Amikacin, Gentamicin, Kanamycin, Neomycin, Netilmicin, Tobramycin, Paromomycin, Geldanamycin, Herbimycin, Loracarbef, Ertapenem, Doripenem, Imipenem/Cilastatin, Meropenem, Cefadroxil, Cefazolin, Cefalotin, Cefalexin, Cefaclor, Cefamandol, Cefoxitin, Cefprozil, Cefuroxim, Cefditoren, Cefoperazon, Cefotaxim, Cefpodoxim, Ceftazidim, Ceftibuten, Ceftizoxim, Ceftriaxon, Cefepim, Ceftarolinfosamil, Ceftobiprol, Teicoplanin, Vancomycin, Telavancin, Clindamycin, Lincomycin, Daptomycin, Azithromycin, Clarithromycin, Dirithromycin, Erythromycin, Roxithromycin, Troleandomycin, Telithromycin, Spectinomycin, Spiramycin, Aztreonam, Furazolidon, Nitrofurantoin, Amoxicillin, Ampicillin, Azlocillin, Carbenicillin, Cloxacillin, Dicloxacillin, Flucloxacillin, Mezlocillin, Methicillin, Nafcillin, Oxacillin, Penicillin G, Penicillin V, Piperacillin, Temocillin, Ticarcillin, Amoxicillin/Clavulanat, Ampicillin/Sulbactam, Piperacillin/Tazobactam, Ticarcillin/Clavulanat, Bacitracin, Colistin, Polymyxin B, Ciprofloxacin, Enoxacin, Gatifloxacin, Levofloxacin, Lomefloxacin, Moxifloxacin, Nalidixinsäure, Norfloxacin, Ofloxacin, Trovafloxacin, Grepafloxacin, Sparfloxacin, Temafloxacin, Mafenid, Sulfonamidochrysoidin, Sulfacetamid, Sulfadiazin, Silber, Sulfadiazin, Sulfamethizol, Sulfamethoxazol, Sulfanilimid, Sulfasalazin, Sulfisoxazol, Trimethoprim, Trimethoprim-Sulfamethoxazol, Demeclocyclin, Doxycyclin, Minocyclin, Oxytetracyclin, Tetracyclin, Clofazimin, Dapson, Capreomycin, Cycloserin, Ethambutol, Ethionamid, Isoniazid, Pyrazinamid, Rifampicin, Rifabutin, Rifapentin, Streptomycin, Arsphenamin, Chloramphenicol, Fosfomycin, Fusidinsäure, Linezolid, Metronidazol, Mupirocin, Platensimycin, Quinupristin/Dalfopristin, Rifaximin, Thiamphenicol, Tigecyclin, Tinidazol, pharmazeutisch annehmbaren Salzen davon und Mischungen aus zwei oder mehreren davon ausgewählt ist, oder wobei das antiinfektiöse Mittel gegebenenfalls aus der Gruppe bestehend aus Polysacchariden, Chitosan, teilweise acetyliertem Chitosan, Polyglucosamin, Chitosandiolen, Polyolen, Aminoalkoholen und Mischungen aus zwei oder mehreren davon ausgewählt ist, oder wobei das antiinfektiöse Mittel gegebenenfalls aus der Gruppe bestehend aus Chlorhexidin, Biguaniden, kationischen Ammoniumverbindungen, kationischen Ammoniumdendrimeren, Silber, Kupfer, kationischen Spezies und Mischungen aus zwei oder mehreren davon ausgewählt ist, wobei die kationischen Ammoniumverbindungen gegebenenfalls aus der Gruppe bestehend aus Cholin und Cholinderivaten ausgewählt sind.

6. Konstrukt nach einem der Ansprüche 1 bis 5, ferner umfassend Zellen, die an der beschichteten Polymeroberfläche des Konstrukts befestigt sind, wobei die Zellen aus der Gruppe bestehend aus Fibroblasten, Endothelzellen, Keratinozyten, Osteoblasten, Chondroblasten, Chondrozyten, Hepatozyten, Makrophagen, Herzmuskelzellen, glatten Muskelzellen, Skelettmuskelzellen, Sehnenzellen, Bandzellen, Epithelzellen, Stammzellen, neuralen Zellen, PC12-Zellen, neuralen Stützzellen, Schwann-Zellen, radialen Gliazellen, Zellen, die Neurosphären bilden, neuralen Tumorzellen, Glioblastomzellen und Neuroblastomzellen ausgewählt sind, wobei die Fibroblasten gegebenenfalls NIH-3T3-Fibroblasten umfassen.

7. Konstrukt nach Anspruch 6, ferner umfassend eine extrazelluläre Matrix (ECM), die von Zellen sezerniert wird und diesen strukturelle und biochemische Unterstützung bereitstellt.

8. Konstrukt nach Anspruch 7, das dezellularisiert ist, um die von den Zellen sezernierte ECM an dem Konstrukt befestigt zu belassen.

9. Verfahren zum Bilden des Konstrukts nach einem der vorstehenden Ansprüche, umfassend:
a) Bereitstellen einer Polymeroberfläche, die gegenüber einer chemischen Bindung einer anorganischen Haftschicht intrinsisch unreaktiv ist;
b) Oxidieren der Polymeroberfläche, um chemisch zugängliche, reaktive Oxy-, Oxo-, Carbonyl-, Carbonsäure- oder Carboxylat-Funktionsgruppen zu bilden, wodurch eine aktivierte Oberfläche bereitgestellt wird;
c) chemisches Binden einer anorganischen Oxid-Haftschicht mit Oberflächenalkoxidgruppen an die reaktiven Funktionsgruppen der aktivierten Polymeroberfläche; und
d) Binden einer selbstorganisierten Phosphonat-Monoschicht (SAMP) an die Oberflächenalkoxidgruppen der anorganischen Oxid-Haftschicht.

10. Verfahren zum Aktivieren einer Polymersubstratoberfläche und Beschichten der aktivierten Substratpolymeroberfläche mit einer anorganischen Oxid-Haftschicht, umfassend die Schritte:
a) Aktivieren der Polymeroberfläche eines nicht aktivierten Substrats zur chemischen Bindung einer anorganischen Haftschicht durch Oxidieren der Polymeroberfläche, um reaktive Oxy-, Oxo-, Carbonyl-, Carbonsäure- oder Carboxylat-Funktionsgruppen auf der Polymeroberfläche zu bilden und dadurch ein aktiviertes Substrat zu bilden;
b) Bereitstellen einer Beschichtungsmischung, umfassend ein organisches Lösungsmittel, das eine reaktive anorganische Alkoxidverbindung enthält, die in dem Lösungsmittel gelöst und/oder dispergiert ist;
c) Suspendieren des aktivierten Polymersubstrats in der Beschichtungsmischung für eine Zeit und bei einer Temperatur, die ausreichend sind, um die reaktiven Funktionsgruppen mit der anorganischen Alkoxidverbindung reagieren zu lassen und eine anorganische Oxidbeschichtung auf der aktivierten Oberfläche des Polymersubstrats zu bilden, um ein mit einer anorganischen Oxid-Haftschicht beschichtetes Substrat bereitzustellen;
d) gegebenenfalls Entfernen des beschichteten Substrats aus der Beschichtungslösung;
e) gegebenenfalls Spülen mit einem Lösungsmittel, um ein gespültes beschichtetes Substrat bereitzustellen; und
f) gegebenenfalls Erwärmen des gespülten beschichteten Substrats auf 35 bis 40 °C;
g) Binden einer selbstorganisierten Phosphonat-Monoschicht (SAMP) an die Oberflächenalkoxidgruppen der anorganischen Haftschicht,
wobei die anorganische Alkoxidverbindung aus der Gruppe bestehend aus den Alkoxiden von Ti, Zr, Al, Mg, Si, Zn, Mo, Nb, Ta, Sn, W und V ausgewählt ist.

11. Verfahren nach den Ansprüchen 9 bis 10, wobei der Aktivierungsschritt eine chemische Oxidation umfasst, wobei die chemische Oxidation gegebenenfalls eine Behandlung mit einem Oxidationsmittel umfasst, das aus der Gruppe bestehend aus Permanganat, Chlorit, Chromsäure, Chromat, Osmiumtetroxid, Rutheniumtetroxid, Iodat, Persäuren, Peroxiden, Fenton-Reagenz, Bleitetraacetat, Bleitetraacetat/Mn(II), Ozon und Sauerstoff ausgewählt ist, oder wobei der Aktivierungsschritt eine Sauerstoffplasmaentladung oder eine Koronaentladung umfasst.

12. Verfahren nach einem der Ansprüche 10 bis 11, wobei die Beschichtungsschritte durch Dampfabscheiden eines anorganischen Alkoxids auf der Oberfläche des aktivierten Substrats und Reagieren der reaktiven Funktionsgruppen mit dem anorganischen Alkoxid ersetzt werden, um eine daran gebundene anorganische Oxid-Haftschicht zu bilden.

13. Konstrukt nach einem der Ansprüche 1 bis 4 oder 6 bis 8, wobei die SAMP kovalent an einen organischen Rest gebunden ist, der aus der Gruppe bestehend aus elektrochemisch aktiven Resten, photochemisch aktiven Resten, zellanziehenden Resten, zelladhäsiven Resten und antiinfektiösen Resten ausgewählt ist.

## Revendications

1. Construction pour la croissance cellulaire, la médecine reconstructive ou des dispositifs médicaux comprenant :
a) une surface de polymère oxydée comprenant des groupes fonctionnels oxy, oxo, carbonyle, acide carboxylique ou carboxylate chimiquement accessibles et réactifs, réactifs avec des alcoxydes inorganiques ; et
b) une couche d'adhérence d'oxyde inorganique liée chimiquement auxdits groupes fonctionnels réactifs de ladite surface de polymère, ladite couche d'adhérence d'oxyde inorganique comprenant des groupes alcoxydes de surface ; et
c) une monocouche auto-assemblée de phosphonate (SAMP) liée aux groupes alcoxydes de surface de ladite couche d'adhérence d'oxyde inorganique,
dans laquelle la surface du polymère, avant oxydation, ne présente pas de groupes fonctionnels de surface exposés en une densité ou une réactivité suffisante pour former des liaisons covalentes ou de coordination avec des alcoxydes inorganiques.

2. Construction selon la revendication 1, dans laquelle ladite surface de polymère comprend un polymère choisi dans le groupe constitué de polyalcanes, polysiloxanes, polyalkylarènes, polyoléfines, polythiols et polyphosphines.

3. Construction selon la revendication 1 ou la revendication 2, dans laquelle ladite couche d'adhérence d'oxyde inorganique comprend un oxyde inorganique choisi dans le groupe constitué des oxydes de Ti, Zr, Al, Mg, Si, Zn, Mo, Nb, Ta, Sn, W, V, et des mélanges de deux ou plus de ceux-ci.

4. Construction selon l'une quelconque des revendications 1 à 3, dans laquelle lesdits phosphonates sont choisis dans le groupe constitué de phosphonates hydrophobes et de phosphonates d'adhésion cellulaire, éventuellement dans laquelle lesdits phosphonates hydrophobes et d'adhésion cellulaire sont choisis dans le groupe constitué d'acides phosphoniques de structure 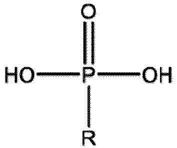 dans laquelle le groupe R est choisi dans le groupe constitué d'un alkyle éventuellement substitué, d'un hétéroalkyle éventuellement substitué, d'un alcényle éventuellement substitué, d'un hétéroalcényle éventuellement substitué, d'un alcynyle éventuellement substitué, d'un hétéroalcynyle éventuellement substitué, d'un aryle éventuellement substitué, d'un arylalkyle éventuellement substitué, d'un hétéroaryle éventuellement substitué et d'un hétéroarylalkyle éventuellement substitué, où l'hétéroalkyle, l'hétéroalcényle, l'hétéroalcynyle, l'hétéroaryle et l'hétéroarylalkyle contiennent un ou plusieurs hétéroatomes choisis dans le groupe constitué par O, N et S, éventuellement dans laquelle lesdits phosphonates hydrophobes sont choisis dans le groupe constitué par R = alkyle en C₃-C₃₀, et lesdits phosphonates d'adhésion cellulaire sont choisis dans le groupe constitué de R = alkyle en C₃-C₃₀ substitué par un groupe phosphonate supplémentaire, éventuellement dans laquelle lesdits phosphonates d'adhésion cellulaire sont choisis dans le groupe constitué de α,ω-diphosphonates en C₃-C₃₀, éventuellement dans laquelle ledit groupe R est le dodécyl-9-ynyle, en outre couplé au niveau du groupe fonctionnel alcyne à l'aide d'une réaction catalysée par un métal, ou éventuellement où ledit groupe R est le dodécyl-9-ynyle, en outre couplé au niveau du groupe fonctionnel alcyne à l'aide d'une réaction.

5. Construction selon l'une quelconque des revendications 1 à 4, dans laquelle ladite SAMP comprend en outre un agent anti-infectieux ou un agent antithrombogène, par exemple l'héparine, lié de manière covalente à celle-ci, éventuellement dans laquelle ledit agent anti-infectieux est un antimicrobien choisi dans le groupe constitué d'amikacine, gentamicine, kanamycine, néomycine, nétilmicine, tobramycine, paromomycine, geldanamycine, herbimycine, loracarbef, ertapénème, doripénème, imipénème/cilastatine, méropénème, céfadroxil, céfazoline, céfalotine, céfalexine, céfaclor, céfamandole, céfoxitine, céfprozil, céfuroxime, céfditorène, céfopérazone, céfotaxime, cefpodoxime, ceftazidime, ceftibutène, ceftizoxime, ceftriaxone, céfépime, ceftaroline fosamil, ceftobiprole, téicoplanine, vancomycine, télavancine, clindamycine, lincomycine, daptomycine, azithromycine, clarithromycine, dirithromycine, érythromycine, roxithromycine, troléandomycine, télithromycine, spectinomycine, spiramycine, aztréonam, furazolidone, nitrofurantoïne, amoxicilline, ampicilline, azlocilline, carbénicilline, cloxacilline, dicloxacilline, flucloxacilline, mezlocilline, méticilline, nafcilline, oxacilline, pénicilline G, pénicilline V, pipéracilline, témocilline, ticarcilline, amoxicilline/acide clavulanique, ampicilline/sulbactam, pipéracilline/tazobactam, ticarcilline/acide clavulanique, bacitracine, colistine, polymyxine B, ciprofloxacine, énoxacine, gatifloxacine, lévofloxacine, loméfloxacine, moxifloxacine, acide nalidixique, norfloxacine, ofloxacine, trovafloxacine, grépafloxacine, sparfloxacine, témafloxacine, mafénide, sulfonamidochrysoïdine, sulfacétamide, sulfadiazine, argent, sulfadiazine, sulfaméthizole, sulfaméthoxazole, sulfanilimide, sulfasalazine, sulfisoxazole, triméthoprime, triméthoprime-sulfaméthoxazole, déméclocycline, doxycycline, minocycline, oxytétracycline, tétracycline, clofazimine, dapsone, capréomycine, cyclosérine, éthambutol, éthionamide, isoniazide, pyrazinamide, rifampicine, rifabutine, rifapentine, streptomycine, arsphénamine, chloramphénicol, fosfomycine, acide fusidique, linézolide, métronidazole, mupirocine, platensimycine, quinupristine/dalfopristine, rifaximine, thiamphénicol, tigécycline, tinidazole, leurs sels pharmaceutiquement acceptables, et des mélanges de deux ou plus de ceux-ci, ou éventuellement dans laquelle ledit agent anti-infectieux est choisi dans le groupe constitué de polysaccharides, chitosane, chitosane partiellement acétylé, polyglucosamine, diols de chitosane, polyols, aminoalcools et des mélanges de deux ou plus de ceux-ci, ou éventuellement dans laquelle ledit agent anti-infectieux est choisi dans le groupe constitué de chlorhexidine, biguanides,composés d'ammonium cationiques, dendrimères d'ammonium cationiques, argent, cuivre, espèces cationiques et mélanges de deux ou plus de ceux-ci, éventuellement dans laquelle lesdits composés d'ammonium cationique sont choisis dans le groupe constitué de choline et des dérivés de choline.

6. Construction selon l'une quelconque des revendications 1 à 5, comprenant en outre des cellules fixées à la surface polymère revêtue de ladite construction, dans laquelle lesdites cellules sont choisies dans le groupe constitué de fibroblastes, cellules endothéliales, kératinocytes, ostéoblastes, chondroblastes, chondrocytes, hépatocytes, macrophages, cellules de muscle cardiaque, cellules de muscle lisse, cellules de muscle squelettique, cellules tendineuses, cellules ligamentaires, cellules épithéliales, cellules souches, cellules neurales, cellules PC12, cellules de soutien neural, cellules de Schwann, cellules gliales radiales, cellules qui forment des neurosphères, cellules tumorales neurales, cellules de glioblastome et cellules de neuroblastome, éventuellement dans laquelle lesdits fibroblastes comprennent des fibroblastes NIH 3T3.

7. Construction selon la revendication 6, comprenant en outre une matrice extracellulaire (MEC) sécrétée par des cellules et fournissant un support structurel et biochimique à celles-ci.

8. Construction selon la revendication 7, qui est décellularisée pour laisser la MEC sécrétée par les cellules fixée à la construction.

9. Procédé de formation de la construction selon l'une quelconque des revendications précédentes, comprenant :
a) la fourniture d'une surface polymère qui est intrinsèquement non réactive vis-à-vis de la liaison chimique d'une couche d'adhérence inorganique ;
b) l'oxydation de la surface de polymère pour former des groupes fonctionnels oxy, oxo, carbonyle, acide carboxylique ou carboxylate chimiquement accessibles, réactifs, fournissant ainsi une surface activée ;
c) la liaison chimique d'une couche d'adhérence d'oxyde inorganique présentant des groupes alcoxydes de surface auxdits groupes fonctionnels réactifs de ladite surface de polymère activée ; et
d) la liaison d'une monocouche auto-assemblée de phosphonate (SAMP) aux groupes alcoxydes de surface de ladite couche d'adhérence d'oxyde inorganique.

10. Procédé d'activation d'une surface de substrat polymère et de revêtement de ladite surface de substrat polymère activé avec une couche d'adhérence d'oxyde inorganique, comprenant les étapes consistant à :
a) activer la surface de substrat polymère non activé pour une liaison chimique d'une couche d'adhérence inorganique par oxydation de ladite surface de polymère pour former des groupes fonctionnels oxy, oxo, carbonyle, acide carboxylique ou carboxylate réactifs sur ladite surface de polymère et former ainsi un substrat activé ;
b) fournir un mélange de revêtement comprenant un solvant organique contenant un composé alcoxyde inorganique réactif qui est dissous et/ou dispersé dans le solvant ;
c) mettre en suspension ledit substrat polymère activé dans ledit mélange de revêtement pendant une durée et à une température suffisantes pour faire réagir lesdits groupes fonctionnels réactifs avec ledit composé alcoxyde inorganique et former un revêtement d'oxyde inorganique sur la surface activée dudit substrat polymère, pour obtenir un substrat revêtu d'une couche d'adhérence d'oxyde inorganique ;
d) éventuellement, retirer le substrat revêtu de la solution de revêtement ;
e) éventuellement, rincer avec un solvant pour obtenir un substrat revêtu rincé ; et
f) éventuellement, chauffer le substrat revêtu rincé à une température de 35 à 40 °C ;
g) lier une monocouche auto-assemblée de phosphonate (SAMP) aux groupes alcoxydes de surface de ladite couche d'adhérence inorganique,
dans lequel ledit composé alcoxyde inorganique est choisi dans le groupe constitué des alcoxydes de Ti, Zr, Al, Mg, Si, Zn, Mo, Nb, Ta, Sn, W et V.

11. Procédé selon les revendications 9 à 10, dans lequel ladite étape d'activation comprend une oxydation chimique, éventuellement dans lequel ladite oxydation chimique comprend un traitement avec un agent oxydant choisi dans le groupe constitué de permanganate, chlorite, acide chromique, chromate, tétroxyde d'osmium, tétroxyde de ruthénium, iodate, peracides, peroxydes, réactif de Fenton, tétra-acétate de plomb, tétra-acétate de plomb/Mn(II), ozone et oxygène, ou dans lequel ladite étape d'activation comprend une décharge de plasma d'oxygène, ou une décharge corona.

12. Procédé selon l'une quelconque des revendications 10 à 11, dans lequel les étapes de revêtement sont remplacées par le dépôt en phase vapeur d'un alcoxyde inorganique sur la surface dudit substrat activé, et la réaction desdits groupes fonctionnels réactifs avec ledit alcoxyde inorganique pour former une couche d'adhérence d'oxyde inorganique liée à celle-ci.

13. Construction selon l'une quelconque des revendications 1 à 4 ou 6 à 8, dans laquelle la SAMP est liée de manière covalente à un groupement organique choisi dans le groupe constitué de groupements électrochimiquement actifs, groupements photochimiquement actifs, groupements attirant les cellules, groupements d'adhésion cellulaire et groupements anti-infectieux.
